# EUROPEAN PATENT SPECIFICATION

(11) **EP 1 830 786 B1**
(45) Date of publication and mention of the grant of the patent: **08.09.2010**
(21) Application number: 05825918.5
(22) Date of filing: 10.11.2005
(51) Int. Cl.: A61K 6/083, A61K 6/09

(54) **WAX-LIKE POLYMERISABLE DENTAL MATERIAL**
WACHSARTIGES POLYMERISIERBARES ZAHNMATERIAL
MATERIAU DENTAIRE POLYMERISABLE DE TYPE CIRE

(30) Priority: 12.11.2004 US 627199 P
(43) Date of publication of application: 12.09.2007
(73) Proprietor: DENTSPLY International Inc., York, PA 17405-0872 (US)
(72) Inventor: SUN, Benjamin, Jiemin, York, Pennsylvania 17402 (US); LICHKUS, Andrew, Murray, York, Pennsylvania 17404 (US)
(74) Representative: Hartz, Nikolai
(86) International application number: PCT/US2005/040980
(87) International publication number: WO 2006/053237

(56) References cited:
- WO-A-02/26197
- WO-A-2006/002086
- US-A- 6 057 383
- US-A1- 2003 113 689
- US-A1- 2004 084 792

## Description

### Technical Field

The invention relates to wax-like polymerizable materials. This wax-like polymerizable dental material is quickly and easily reshaped. The shaped wax-like polymerizable dental material is cured to form dental products, such as crowns, bridges, dentures, and other restoration devices and appliances.

### Background of the Invention

US 2003/0113689 discloses a dental polymeric material formed form a wax-like polymerisable material. A low shrinkage and low stress dental material is known from WO 2006/002086. Volkel et al in US Patent 6,057,383 (and Canadian Patent Application 2207351), assigned to lvoclar, disclose wax-like polymerizable material for making entire dental products. The prior art does not disclose a wax-like polymerizable material for forming dentures or other high toughness products.

### Detailed Description of the Invention

The invention provides wax-like polymerizable dental material and restorative paste wax polymerizable dental material. Specifically, the present invention provides a polymerisable dental material having resistance to shrinkage, which is defined by claim 1. Wax-like polymerizable dental materials of this invention have low polymerization shrinkage. The polymerization of wax-like material of this invention involves ring opening polymerization as well as volume expansion from phase change to reduce the polymerization shrinkage. Previous epoxy-based resins do not have wax-like handling characteristics and not involve volume expansion from phase change. Wax-like polymerizable dental material is flowable at and above 40°C, and becomes dimensionally stable at and below 23°C, within 5 minutes. Paste wax material is flowable at and above 50°C, and becomes dimensionally stable at and below 37°C, within 5 minutes. These polymerizable dental materials may include filler particles, fiber and/or rubber-modified high molecular weight resin. These polymerizable dental materials are useful in tooth restorative fillings, adhesives, cements, denture base materials, orthodontic materials and sealants, for repair of defects in natural dentition, and to form crowns, bridges, full dentures, partial dentures, denture liners, custom trays, artificial teeth, repairs for natural teeth, veneers, denture repairs, denture reline, night guards, splints, retainers, orthodontic components, burn out parts, provisional dental devices, inlays, onlays, orthodontic appliances, oral orthopedic appliances, temporary dentures, temporary partial dentures, maxillofacial prostheses, obturators, and occular prostheses, etc.

Polymerizable dental material in accordance with the invention may include from 0 to about 95 percent by weight filler particles. In a preferred embodiment of the invention polymerizable dental materials include from about 5 to about 90 percent by weight filler. More preferably, these polymerizable dental materials include from about 20 to about 85 percent by weight filler. Most preferably, these polymerizable dental materials include from about 40 to about 80 percent by weight filler.

The filler particles have a range of particles sizes from 0.001 micrometers to 10 micrometers. The filler particles preferably include organic and/or inorganic particles, and preferably reduce polymerization shrinkage, improve wear resistance and modify the mechanical and physical properties. Preferred fillers are glasses formed from or including, barium, calcium, strontium, lanthanum, tantalum, and/or tungsten silicates and aluminates and/or aluminosilicates, silica, quartz, ceramics, nanoparticles. Peferably the filler particles have a range of particle sizes of from 0.001 micrometers to 10 micrometers.

The polymerizable dental materials of the invention are quickly and easily reshaped, for example by warming, and shaping it while warm and then allowing it to cool to body (37°C) or room temperature (23°C). The cooled polymerizable dental materials may be worked for example by packing, molding, shaping, and/or carving. The worked polymerizable dental materials are cured.

The polymerizable dental materials of the invention preferably include from about 1 to about 100 percent by weight of a crystalline resin and from about 0 to 99 percent by weight of an amorphous component. When heated, the polymerizable dental materials soften and are more flowable and less crystalline.

Wax-like polymerizable dental material and restorative paste wax polymerizable dental material of the invention may include pigments, initiators, catalysts, stabilizers, plasticizers and fibers. Preferred stabilizers are butylated hydroxytoluene (BHT) and the methyl ether of hydroquinone (MEHQ).

Polymerizable dental materials of the invention may include one or more initiating systems to cause them to harden promptly. Light curable wax-like polymerizable dental composites preferably include at least a light sensitizer, for example camphorquinone, 4-octyloxy-phenyl-phenyl iodonium hexafluoroantimonate (OPPI), 2,4,6- trimethylbenzoyldiphenylphosphine oxide, or methyl benzoin which causes polymerization to be initiated upon exposure to activating wavelengths of light; and/or a reducing compound, for example tertiary amine.

A room temperature or heat activating catalyst system is preferably included in polymerizable dental materials of the invention. For example a peroxide capable of producing free radicals when activated by a reducing agent at room temperature or by heating. Preferred peroxides include benzyl peroxide and lauroyl peroxide.

Compounds, which are readily partially crystallizable and useful in wax-like polymerizable dental material of a preferred embodiment of the invention, include epoxy, epoxy methacrylate (or acrylate), methacrylate (or acrylate) compounds or their combinations. Epoxy compounds polymerizes by ring-opening polymerization shrinks less due to the increase in excluded free-volume associated with the ring-opening process in addition to the volume expansion from the phase conversion.

Polymerizable dental materials of the invention are preferably rapidly partially recrystallizable. Rapid recrystallizability provides the densification of the polymeric products and a combination of flowability and dimensional stability, depending on its temperature prior to polymerization. When polymerized, the crystallized phase melts effective resulting in volume expansion, which offsets polymerization shrinkage. The combination of volume expansion from phase change and ring-opening process ensures the low polymerization shrinkage or even polymerization expansion. Thus, the polymeric products are low shrinkage and low stress restorations and devices. "Crystallinity" as used herein refers to regularity and order within a material resulting in a heat of fusion of at least 1.0 J/g at and below 50°C. Heat of Fusion as used herein refers to enthalpy of fusion as determined by ASTM 793-95. Percent crystallinity is determined by measuring the heat of fusion using differential scanning calorimetry according to ASTM test method E 793-95.

A preferred embodiment of the invention provides a dental polymeric material formed by light curing wax-like polymerizable dental material and restorative paste wax polymerizable dental material. "Flexural strength, and flexural modulus" as used herein refers to results of testing according to ASTM D790 (1997).

"Wax-like" as used herein refers to material which is flowable (fluid) at and above 40°C, and becomes dimensionally stable (solidifies: i.e. is non-fluid) at least at and below 23°C, within 5 minutes. Thus, wax-like material is flowable when it is at and above 40°C, and becomes dimensionally stable when it is at and below 23°C. Flowable wax-like material having a temperature from 100°C to 40°C, becomes dimensionally stable within 5 minutes by cooling by exposure to an ambient temperature between 37°C and 0°C. Flowable wax-like composite paste having a temperature from 100°C to 40°C, becomes dimensionally stable within (in order of increasing preference) 4, 2, 1 or 0.5 minutes by cooling by exposure to an ambient temperature between 23°C and 0°C.

"Restorative Paste Wax" as used herein refers to material which is flowable (fluid) at and above 50°C, and becomes dimensionally stable (solidifies: i.e. is non-fluid) at least at and below 37°C, within 5 minutes. Thus, restorative paste wax is flowable when it is at and above 50°C, and becomes dimensionally stable when it is at and below 37°C. Flowable restorative paste wax having a temperature from 100°C to 50°C, becomes dimensionally stable within 5 minutes by cooling by exposure to an ambient temperature between 37°C and 0°C. Flowable restorative paste wax having a temperature from 100°C to 50°C, becomes dimensionally stable within (in order of increasing preference) 4, 2, 1 or 0.5 minutes by cooling by exposure to an ambient temperature between 37°C and 0°C. Restorative paste wax may be flowable throughout all of the temperature range from 49°C to 38°C; it may be dimensionally stable throughout all of the temperature range from 49°C to 38°C; or it may be flowable in part and dimensionally stable in part of the temperature range from 49°C to 38°C.

Dimensional stability is determined by testing according to ADA (American Dental Association) consistency test specification 19, paragraph 4.3.4, JAVA Vol. 94, April, 1977, pages 734-737 at 23°C. Fluids change shape uniformly in response to external force imposed on them (see Hawley's Condensed Chemical Dictionary 1997, page 507, at fluid).

In order of increasing preference polymerization shrinkage of wax-like polymerizable dental material and restorative paste wax polymerizable dental material of the invention is less than 3 percent by volume, less than 2 percent by volume, less than 1.5 percent by volume, less than 1 percent by volume, less than 0.5 percent by volume. In order of increasing preference polymerization shrinkage of restorative paste wax polymerizable dental material of the invention is less than 3 percent by volume, less than 2 percent by volume, less than 1.5 percent by volume, less than 1 percent by volume, less than 0.5 percent by volume.

A preferred embodiment of the invention provides a prepared cavity in a tooth in a patient's mouth, which is then filled by injection from a syringe of dental filling material in accordance with the invention. Preferably the syringe is heated to from 42°C to 60°C, and has a readily disconnected and interchangeable nozzle with a generally cylindrical internal passage having an internal diameter of from about 0.5 mm to about 5.0 mm. The dental filling material cools and solidifies rapidly in the prepared cavity in the tooth to about 37°C. Thus, a syringe is provided having an inner chamber and a nozzle. The nozzle has a nozzle passage in fluid flow communication with the inner chamber. The inner chamber encloses wax-like polymerizable dental material or restorative paste wax polymerizable dental material. Then the polymerizable dental material is polymerized.

A preferred embodiment of the invention provides a prepared cavity in a tooth in a patient's mouth, which is then filled by positioning in the prepared cavity a composition including at least 40 percent by weight filler and a polymerizable dental material selected from the group consisting of wax-like polymerizable dental material and restorative paste wax polymerizable dental material. Then the polymerizable dental material is light cured to form dental polymeric material with a shrinkage during polymerization of less than 2 percent by volume. The polymerizable dental material includes a portion of crystals, which melt during polymerization. The crystals are believed to be crystals of oligomer and/or crystals of monomer. The volume of the liquid formed by melting the crystals is greater than the volume of the crystals. This expansion reduces the shrinkage of the polymerizable dental material caused by polymerization. In addition, the wax-like polymerizable materials of this invention showed low polymerization shrinkage due to the ring opening process involved. This invention provides low polymerization shrinkage materials with and without fillers. Furthermore, low polymerization shrinkage materials offer low stress restorations and low stressed cured dental devices.

Moreover, the compositions of this invention provide a system with two types of initiators to initiate rapid free radical polymerization and relatively slower cationic polymerization. The incorporation of cationic polymerization slows down the complete polymerization conversion process, so as to reduce polymerization stress and more effective introduction of stress reduction by volume expansion from melting phase change and ring opening process.

In the following examples, unless otherwise indicated, all parts and percentages are by weight; Lucirin TPO refers to 2,4,6-trimethylbenzoyldiphenylphosphine oxide and liquid Lucirin refers to ethyl-2,4,6-trimethylbenzoylphenylphosphinate made by BASF.

### EXAMPLE 1A

### Preparation of Epoxy Oligomer

Epoxy oligomer was prepared by mixing of 0.2 gram of 2-methylimidazole and 41.25 grams of bis(3,4-epoxy-6-methylcyclohexylmethyl)adipate, and 26.0 grams of 1,10 decanediol. Then the mixture is stirred under nitrogen for 8 hours at temperatures between 120 to 150°C. After the reaction, resin is collected as yellow liquid and cooled down to room temperature to form gel-like waxy solid.

### EXAMPLE 1B

### Preparation of Epoxy Oligomer

Epoxy oligomer I was prepared by mixing of 0.12 gram of 2-methylimidazole and 29.3 grams of bisphenol A proxylate diglycidyl ether, 16.2 grams of 1,10-decanediol in a three-neck flask. Then the mixture is stirred under nitrogen for 8 hours at temperatures from 100 to 150°C. After the reaction, light yellow resin is collected and cooled down to room temperature to form soft wax-like resin.

### EXAMPLE 2A

### Preparation of Monomer

A reaction flask was charged with 700 grams of 1,6-diisocyanatohexane and heated to about 70°C under a positive nitrogen pressure. To this reactor were added 1027 grams of 2-hydroxyethyl methacrylate, 0.75 gram of catalyst dibutyltin dilaurate and 4.5 grams of butylated hydroxy toluene (BHT). The addition was slow and under dry nitrogen flow over a period of two hours. The temperature of the reaction mixture was maintained between 70°C and 90°C for another two hours and followed by the addition of 8.5 grams of purified water. One hour later, the reaction product was discharged as clear liquid into plastic containers and cooled to form a white solid and stored in a dry atmosphere.

### EXAMPLE 2B

### Preparation of Monomer

A reaction flask was charged with 43.8 grams of 1,12-diisocyanatododecane and heated to about 80°C under a positive nitrogen pressure. To this reactor were added 45.0 grams of 2-hydroxyethyl methacrylate, 0.075 gram of catalyst dibutyltin dilaurate and 0.19 grams of butylated hydroxy toluene (BHT). The addition was slow and under dry nitrogen flow over a period of one hour. The temperature of the reaction mixture was maintained between 80°C and 90°C for another one hour and followed by the addition of 0.15 gram of purified water. One hour later, the reaction product was discharged as clear liquid into plastic container and cooled to form a white solid and stored in a dry atmosphere.

### EXAMPLE 3

### Preparation of Epoxy Oligomer

Epoxy oligomer is prepared by mixing of 1 part 2-methylimidazole and 59 parts of bisphenol A propoxylate diglycidyl ether, 40 parts of bisphenol A propoxylate. Then the mixture is stirred under nitrogen for 8 hours at three different temperatures: 140, 160 and 180°C in a reactor. After the reaction, semi-solid resin is collected.

### EXAMPLE 4

### Preparation of Polymerizable Denture Setup Material

A wax-like polymerizable dental material was prepared by stirring at 85°C a liquid mixture of 68.25 grams of bisphenol A proxylate diglycidyl ether, 30.0 grams of 1, 10-decanediol, 1.0 gram 4-octyloxy-phenyl iodonium hexafluoroantimonate (OPPI), 0.35 gram of 2,4,6-trimethylbenzoyldiphenylphosphine oxide, (Lucirin TPO made by BASF), 0.1 gram of red acetate fibers, 0.3 gram of pigment concentrates.

### EXAMPLE 5

### Preparation of Polymerizable Denture Contour Material

A wax-like polymerizable dental material was prepared by stirring at 85°C a liquid mixture of 48.25 grams of bisphenol A proxylate diglycidyl ether, 50.0 grams of polycaprolactone diol (average Mn, - 2000), 1.0 gram 4-octyloxy-phenyl iodonium hexafluoroantimonate (OPPI), 0.35 gram of 2,4,6-trimethylbenzoyldiphenylphosphine oxide, (Lucirin TPO made by BASF), 0.1 gram of red acetate fibers, 0.3 gram of pigment concentrates.

### EXAMPLES 6A through 6D

Table 1 shows the components of the compositions of Examples 6A through 6D. The compositions of Examples 6A through 6D were prepared by mixing the components shown in Table 1 at 90°C.

**TABLE 1**

| Components | 6A | 6B | 6C | 6D |
|---|---|---|---|---|
| Camphorquinone (CQ) | 0.17 | 0.17 | 0.15 | 0.2 |
| 4-Octyloxy-phenyl-phenyl iodonium hexafluoroantimonate (OPPI) | 0.33 | 0.33 | 0.3 | 0.4 |
| Bisphenol A proxylate diglycidyl ether | 23.5 | | | |
| 3,4-Epoxycyclohexyl methyl 3,4-epoxycyclohexanecarboxylate | | 19.5 | 16.55 | |
| Bis(3,4-epoxycyclohexylmethyl)adipate | | | | 29.4 |
| 1,10-decanediol | 6 | | 3 | 5 |
| Polycaprolactone (Mn - 2000) | | | 5 | 5 |
| 2,5-Dimethyl-2,5-hexandiol | | 5 | | |
| Silanated barium aluminoflurosilicate glass (BAFG) * (EG) | 14 | 15 | 15 | 11 |
| Silanated barium aluminoflurosilicate glass (BAFG) ** (U/F) | 56 | 60 | 60 | 49 |

| | | | | |
|---|---|---|---|---|
| * particles having an average particle size of from about 1 to about 10 micrometers. ** particles having an average particle size of from about 0.1 to about 1 micrometers. | | | | |

### EXAMPLE 7

### Filling Material

A cavity in a natural tooth in a patient's mouth is prepared by drilling, and then brushing onto the drilled cavity about 0.02 ml of PRIME & BOND NT dual cure bonding agent, sold by Dentsply International Inc. Then the bonding agent is light cured by impinging light thereon for 30 seconds from a Spectrum 800 light curing unit sold by Dentsply International Inc. The prepared cavity is then filled with 0.2 g of the product of Example 6A, which is then light cured by impinging light thereon for 30 seconds from a Spectrum 800 light curing unit sold by Dentsply International Inc.

### EXAMPLE 8A

### Crown

A crown is formed by molding about 0.5 g of the product of Example 6B. A surface of natural tooth in a patient's mouth is prepared for the crown by cutting and polishing, and then brushing onto the polished surface about 0.05 ml of PRIME & BOND NT dual cure bonding agent, sold by Dentsply International Inc. Then the crown is set onto the prepared surface: The crown and the bonding agent are then light cured by impinging light thereon for 60 seconds from a Spectrum 800 light curing unit sold by Dentsply International Inc.

### EXAMPLE 8B

### Veneer

A veneer is formed by molding about 0.3 g of the product of Example 6C. A surface of natural tooth in a patient's mouth is prepared for the veneer by cutting and polishing, and then brushing onto the polished surface 0.03 ml of PRIME & BOND NT dual cure bonding agent, sold by Dentsply International Inc. Then the veneer is set onto the prepared surface. The veneer and the bonding agent are then light cured by impinging light thereon for 60 seconds from a Spectrum 800 light curing unit sold by Dentsply International Inc.

### EXAMPLE 8C

### Filling Material

A natural dentition in a patient's mouth in need of restoration is selected. A cavity in the tooth is prepared by drilling, and then brushing onto the drilled cavity 0.02 ml of PRIME & BOND NT dual cure bonding agent, sold by Dentsply International Inc. Then the bonding agent is light cured by impinging light thereon for 30 seconds from a Spectrum 800 light curing unit sold by Dentsply International Inc. The prepared cavity is then filled by injection into the cavity of 0.2 g of the product of Example 6D from a syringe having a nozzle with an internal passage diameter of about 2 mm. The syringe is warmed to 50°C, and has a chamber filled with the dental filling material product of Example 6D. The dental filling material cools to 37°C and solidifies rapidly with excellent shape stability. The cooled filling material is carved and sculptured to conform to the contour and shape of the tooth. The cooled dental filling material is then light cured by impinging light thereon for 30 seconds from a Spectrum 800 light curing unit sold by Dentsply International Inc.

### EXAMPLE 9

### Green Tooth

A tooth is formed by molding 0.6 g of the product of Example 6C into the shape of a natural tooth.

### EXAMPLE 10

### High Strength Tooth

The tooth formed in Example 9 is light cured by impinging light thereon for 10 minutes from an Eclipse light curing unit sold by Dentsply International Inc. A high strength polymeric artificial tooth is formed which has a polymerization shrinkage of less than 2 percent by volume.

It should be understood that while the present invention has been described in considerable detail with respect to certain specific embodiments thereof, it should not be considered limited to such embodiments but may be used in other ways without departure from the scope of the appended claims.

## Claims

1. A polymerizable dental material having resistance to shrinkage, comprising
(i) a crystallizable material that can be melted and which exhibits a volume expansion upon melting, said material is formed from an epoxy oligomer which can be initiated to undergo ring open polymerization, and
(ii) a system with two types of initiators to initiate rapid free radical polymerization and relatively slower cationic polymerization.

2. A dental material as in claim 1 , wherein said crystallizable material is flowable at temperatures above 40 °C and which is dimensionally stable below 23 °C.

3. A dental material as in claim 1 , wherein said crystallizable material is wax-like.

4. A dental material as in claim 1, wherein said epoxy oligomer is the reaction product of an imidazole and an adipate.

5. A dental material as in claim 4, wherein said imidazole is 2-methylimidazole.

6. A dental material as in claim 4, wherein said adipate is bis(3,4-epoxy-6-methylcyclohexylmethyl)adipate.

7. A dental material as in claim 4, wherein said reaction product is also reacted in the presence of a diol.

8. A dental material as in claim 7, wherein said diol is 1 ,10-decanediol.

9. A dental material as in claim 1, wherein said epoxy oligomer is the reaction product of an imidazole, bisphenol A propoxylate diglycidyl ether and a diol.

10. A dental material as in claim 9, wherein said imidazole is 2-methylimidazole.

11. A dental material as in claim 9 , wherein said diol is 1,10-decanediol.

## Patentansprüche

1. Polymerisierbares Dentalmaterial mit Schrumpfungsresistenz, umfassend
(i) ein kristallisierbares Material, das geschmolzen werden kann und das beim Schmelzen eine Volumenexpansion zeigt, wobei das Material erzeugt wird aus einem Epoxyoligomer, das initiiert werden kann und eine Ringöffnungspolymerisation eingeht, und
(ii) ein System mit zwei Typen von Initiatoren, um eine schnelle Radikalpolymerisation und eine relativ langsamere kationische Polymerisation zu initiieren.

2. Das Dentalmaterial nach Anspruch 1, wobei das kristallisierbare Material bei Temperaturen von über 40°C fließfähig ist, und das unterhalb von 23°C dimensionsstabil ist.

3. Das Dentalmaterial nach Anspruch 1, wobei das kristallisierbare Material wachsartig ist.

4. Das Dentalmaterial nach Anspruch 1, wobei das Epoxyoligomer ein Reaktionsprodukt aus einem Imidazol und einem Adipat ist.

5. Das Dentalmaterial nach Anspruch 4, wobei das Imidazol 2-Methylimidazol ist.

6. Das Dentalmaterial nach Anspruch 4, wobei das Adipat bis(3,4-Epoxy-6-methylcyclohexylmethyl)adipat ist.

7. Das Dentalmaterial nach Anspruch 4, wobei das Reaktionsprodukt auch in Gegenwart eines Diols umgesetzt wird.

8. Das Dentalmaterial nach Anspruch 7, wobei das Diol 1,10-Dekandiol ist.

9. Das Dentalmaterial nach Anspruch 1, wobei das Epoxyoligomer ein Reaktionsprodukt aus einem Imidazol, Bisphenol-A-propoxylatdiglycidylether und einem Diol ist.

10. Das Dentalmaterial nach Anspruch 9, wobei das Imidazol 2-Methylimidazol ist.

11. Das Dentalmaterial nach Anspruch 9, wobei das Diol 1,10-Dekandiol ist.

## Revendications

1. Matière dentaire polymérisable présentant une résistance au retrait, comprenant
(i) une matière cristallisable qui peut être fondue et qui présente une expansion de volume lors de la fusion, ladite matière étant formée à partir d'un oligomère époxy qui peut être soumis à un déclenchement de polymérisation par ouverture de cycle, et
(ii) un système avec deux types d'initiateurs pour déclencher une polymérisation rapide par radicaux libres et une polymérisation cationique relativement plus lente.

2. Matière dentaire suivant la revendication 1, dans laquelle ladite matière cristallisable est apte à l'écoulement à des températures supérieures à 40°C et est dimensionnellement stable à une température inférieure à 23°C.

3. Matière dentaire suivant la revendication 1, dans laquelle ladite matière cristallisable est analogue à une cire.

4. Matière dentaire suivant la revendication 1, dans laquelle ledit oligomère époxy est le produit de réaction d'un imidazole et d'un adipate.

5. Matière dentaire suivant la revendication 4, dans laquelle ledit imidazole est le 2-méthylimidazole.

6. Matière dentaire suivant la revendication 4, dans laquelle ledit adipate est l'adipate de bis(3,4-époxy-6-méthylcyclohexylméthyle).

7. Matière dentaire suivant la revendication 4, dans laquelle ledit produit de réaction est également amené à réagir en présence d'un diol.

8. Matière dentaire suivant la revendication 7, dans laquelle ledit diol est le 1,10-décanediol.

9. Matière dentaire suivant la revendication 1, dans laquelle ledit oligomère époxy est le produit de réaction d'un imidazole, d'éther diglycidylique de propoxylate de bisphénol A et d'un diol.

10. Matière dentaire suivant la revendication 9, dans laquelle ledit imidazole est le 2-méthylimidazole.

11. Matière dentaire suivant la revendication 9, dans laquelle ledit diol est le 1,10-décanediol.
